# FASCICULE DE BREVET EUROPEEN

(11) **EP 2 683 685 B1**
(45) Date de publication et mention de la délivrance du brevet: **27.07.2016**
(21) Numéro de dépôt: 12707602.4
(22) Date de dépôt: 08.03.2012
(51) Int. Cl.: C07C 253/14

(54) **PROCEDE DE PREPARATION DU DIFLUOROACETONITRILE ET DE SES DERIVES**
VERFAHREN ZUR HERSTELLUNG VON DIFLUORACETONITRIL UND DERIVATEN DAVON
METHOD FOR PREPARING DIFLUOROACETONITRILE AND THE DERIVATIVES THEREOF

(30) Priorité: 09.03.2011 FR 1100708
(43) Date de publication de la demande: 15.01.2014
(73) Titulaire: RHODIA OPERATIONS, 75009 Paris (FR)
(72) Inventeur: BUISINE, Olivier, F-69230 Saint-Genis-Laval (FR)
(74) Mandataire: Menville, Laure
(86) Numéro de dépôt international: PCT/EP2012/053959
(87) Numéro de publication internationale: WO 2012/120067

(56) Documents cités:
- WO-A1-2010/142377
- SU-A1- 290 697
- US-A1- 2006 122 399

## Description

La présente invention a pour objet un procédé de préparation du difluoroacétonitrile et de ses dérivés.

Plus précisément, l'invention concerne la préparation du difluoroacétonitrile et de ses dérivés tels que acide, sel, ester ou amide.

Le difluoroacétonitrile est un composé organique fluoré qui est utilisé comme intermédiaire de fabrication dans différents domaines d'application notamment dans les domaines agrochimique et pharmaceutique.

Ainsi, il est souhaitable de disposer d'un procédé de préparation susceptible d'être mis en oeuvre à l'échelle industrielle.

La demande internationale de brevet WO 2010/142377 décrit un procédé de préparation de composés fluoroalkylnitriles par réaction d'amides fluorés avec des halogénures d'acyle, en présence d'une base et d'une quantité catalytique d'acides carboxyliques fluorés.

Le document brevet SU 290697 décrit un procédé de préparation de difluoroacétonitrile par réaction du difluorométhane avec du chlorure de cyanogène à haute température.

Il a maintenant été trouvé et c'est ce qui constitue l'objet de la présente invention, un procédé de préparation de difluoroacétonitrile caractérisé par le fait qu'il comprend la réaction d'un halogénodifluorométhane et d'une source d'anions cyanure, en milieu basique.

Le substrat de départ qui intervient dans le procédé de l'invention répond à la formule suivante :

HXCF₂ (I)

dans ladite formule, X représente un atome de chlore, de brome ou d'iode.

Dans la formule (I), X représente de préférence un atome de chlore ou de brome.

Encore plus préférentiellement, X représente un atome de chlore car le composé correspondant de formule (I) est le chlorodifluorométhane (ou R22) qui est une matière première de choix car fabriqué à grande échelle.

Ce composé est disponible à température ambiante et sous pression atmosphérique, sous forme gazeuse.

Par « source d'anions cyanure », on entend un sel apportant un anion CN⁻.

Comme exemples de sels convenant au procédé de l'invention, on peut citer les sels de métaux alcalins ou alcalino-terreux.

Par « alcalin » et « alcalino-terreux », on fait référence aux éléments des groupes (IA) et (IIA) de la classification périodique des éléments.

Dans le présent texte, on se réfère ci-après à la Classification périodique des éléments publiée dans le Bulletin de la Société Chimique de France, n°1 (1966).

Pour des raisons économiques évidentes, on choisit plus particulièrement le cyanure de sodium et de potassium. Toutefois, l'invention n'exclut pas toute autre source d'anions cyanure.

La concentration de la source de cyanure exprimée en CN⁻ peut représenter de 5 à 40 % du poids de l'eau.

La quantité de source de cyanure introduite est choisie d'une manière préférentielle de telle sorte que le rapport entre le nombre de moles de source de cyanure exprimée en CN⁻ et le nombre de moles d'halogénodifluorométhane varie entre 0,8 et 2,0 et de préférence entre 0,9 et 1,0.

Conformément au procédé de l'invention, la réaction de cyanation est conduite en présence d'une base.

La caractéristique de la base est qu'elle ait un pka au moins supérieur ou égal à 10, de préférence compris entre 10 et 14.

Le pKa est défini comme la constante de dissociation ionique du couple acide/base, lorsque l'eau est utilisée comme solvant.

Pour le choix d'une base ayant un pKa tel que défini par l'invention, on peut se reporter, entre autres, au Handbook of Chemistry and Physics, 66ème édition, p. D-161 ET D-162.

Selon le procédé de l'invention, la réaction de cyanation est conduite dans un milieu aqueux contenant en solution un agent basique, et plus particulièrement les bases alcalines ou alcalino-terreuses parmi lesquelles on peut citer des hydroxydes tels que l'hydroxyde de sodium, de potassium, de lithium et la baryte ; des alcoolates alcalins tels que le méthylate, l'éthylate, l'isopropylate et le t-butylate de sodium ou de potassium ; des carbonates ou bicarbonates de sodium ou de potassium et de façon générale, les sels des bases alcalines ou alcalino-terreuses et d'acides faibles.

Pour des considérations économiques, on fait appel à l'hydroxyde de sodium ou de potassium.

D'une manière avantageuse, on fait appel à une solution aqueuse d'hydroxyde de métal alcalin ayant une concentration généralement comprise entre 5 et 50 % en poids. La concentration de la solution de départ n'est pas critique. Toutefois, on préfère faire appel à une solution plus concentrée de concentration variant entre 30 et 50 % en poids.

On peut également ajouter par voie séparée, la base sous forme solide et l'eau.

La quantité de base mise en oeuvre préférentiellement est telle que le rapport entre le nombre de moles de base exprimée en OH⁻ et le nombre de moles de chlorodifluorométhane varie de préférence entre 0,05 et 0,5, et plus préférentiellement entre 0,05 et 0,2.

Conformément au procédé de l'invention, la réaction est conduite de préférence en milieu aqueux bien qu'un solvant réactionnel ne soit pas exclus.

L'eau est généralement apportée par la solution basique.

La température de la réaction de cyanation est choisie de manière qu'elle soit suffisante pour permettre l'accomplissement de la réaction.

La température de la réaction est choisie de préférence entre 20°C et 150°C et plus préférentiellement entre 50°C et 120°C.

Elle se déroule généralement sous pression autogène des réactifs et des produits: la pression se situant généralement entre 10 et 150 bar (absolu).

On peut éventuellement conduire la réaction de préférence sous atmosphère d'un gaz inerte qui peut être l'azote ou un gaz rare, de préférence l'argon : l'azote étant préféré notamment compte tenu de son coût réduit.

D'un point de vue pratique, le procédé selon l'invention est simple à mettre en oeuvre.

On mélange la source de cyanure, la solution basique (ou base + eau) puis l'on introduit dans ce milieu réactionnel, l'halogénodifluorométhane.

On porte ensuite l'ensemble à la température réactionnelle choisie dans l'intervalle précitée que l'on maintient par exemple entre 2 et 8 heures.

En fin de réaction, on obtient un milieu biphasique comprenant le difluoroacétonitrile.

Plus précisément, à température ambiante (15°C à 25°C), la phase aqueuse comprend la base et éventuellement l'excès de la source de cyanure en solution aqueuse et la phase organique comprend le difluoroacétonitrile et éventuellement l'excès l'halogénodifluorométhane.

On récupère le difluoroacétonitrile par exemple en séparant par décantation les phases aqueuse et organique et distillation d'une manière classique de la phase organique permettant ainsi d'obtenir le difluoroacétonitrile.

Un autre mode de séparation du difluoroacétonitrile consiste à effectuer la distillation du milieu issu de la réaction sans avoir au préalable effectué une décantation.

L'étape de distillation est conçue pour obtenir :
- en pied de distillation, l'eau, la base et éventuellement l'excès de source d'anions cyanure,
- et en tête de distillation, une phase gazeuse comprenant d'abord l'excès d'halogénodifluorométhane puis le difluoroacétonitrile attendu.

La distillation est conduite à une température dans le bouilleur choisie de manière que la température soit suffisante pour obtenir en tête de colonne, la température d'ébullition du difluoroacétonitrile.

On conduit de préférence la distillation sous pression atmosphérique. Toutefois, une pression légèrement inférieure ou supérieure à la pression atmosphérique est également possible.

La distillation est effectuée à l'aide d'une colonne de distillation classique.

Lorsque la température en tête de colonne est entre 20°C et 22°C, à pression atmosphérique, on recueille en tête de colonne une phase gazeuse constituée du difluoroacétonitrile.

On refroidit cette phase gazeuse et la transforme sous forme liquide par refroidissement à une température par exemple comprise entre -10°C et 15°C, de préférence comprise entre 0°C et 10 °C.

Cette opération est conduite par passage dans un condenseur qui est un appareil classique par exemple un échangeur tubulaire alimenté par de l'eau ou par un fluide maintenu à une température inférieure à la température de refroidissement choisie généralement inférieure de 5°C à 10°C.

Le flux condensé est constitué par le difluoroacétonitrile et une fraction de ce flux peut être introduite, latéralement en tête de colonne de distillation afin d'assurer le reflux de la colonne.

Un autre objet de l'invention consiste à utiliser le difluoroacétonitrile préparé précédemment comme intermédiaire de fabrication.

Ainsi, l'invention concerne également un procédé de préparation de l'acide difluoroacétique, de ses sels, esters ou amide caractérisé par le fait qu'il comprend une première étape de préparation de difluoroacétonitrile à partir d'un halogénodifluorométhane selon le procédé décrit précédemment suivie par une opération d'hydrolyse.

Le procédé de l'invention permet donc de préparer les dérivés répondant à la formule suivante :

HCF₂-COOR₁ (II)

dans ladite formule :
- R₁ représente :
   - un atome d'hydrogène,
   - un groupe hydrocarboné, substitué ou non, qui peut être un groupe alkyle ou cycloalkyle,
   - un cation métallique,
   - un groupe amino.

Selon une première variante de l'invention, on prépare l'acide difluoroacétique qui répond à la formule (II) dans laquelle R₁ représente un atome d'hydrogène.

Le procédé de préparation de l'acide difluoroacétique comprend l'hydrolyse du difluoroacétonitrile obtenu en milieu acide.

La quantité d'eau mise en oeuvre à cette étape d'hydrolyse est généralement telle que le rapport entre le nombre de moles d'eau et le nombre de moles de difluoroacétonitrile est compris entre 2 et 5.

L'acidification est réalisée à l'aide d'un acide fort ayant un pkₐ inférieur ou égal à 2.

Le pKa est défini comme la constante de dissociation ionique du couple acide/base, lorsque l'eau est utilisée comme solvant.

On choisit un acide fort qui avantageusement ne présente pas de caractère oxydant. Ainsi, l'acide nitrique n'est pas préféré. On fait appel plus préférentiellement à l'acide sulfurique, chlorhydrique ou phosphorique.

L'acide sulfurique est choisi préférentiellement.

On fait appel avantageusement à une solution d'acide fort concentrée.

On utilise plus particulièrement les formes commerciales d'acides.

On peut citer notamment les solutions d'acide sulfurique à 95 ou 98 % en masse, d'acide chlorhydrique à 37 % en masse, d'acide phosphorique à 95 - 100 % en masse.

La quantité d'acide fort mis en oeuvre est telle que le rapport entre le nombre de moles d'acide exprimé en ions H⁺ et le nombre de difluoroacétonitrile varie entre 1 et 5, de préférence entre 1 et 2.

L'opération d'hydrolyse est avantageusement conduite à une température comprise entre 100°C et 150°C.

On travaille généralement sous pression autogène des réactifs et produits.

On peut également établir une atmosphère inerte, de préférence une atmosphère d'azote.

Selon un mode de réalisation pratique de l'invention, on mélange le difluoroacétonitrile et la solution acide.

On porte le mélange réactionnel à la température d'hydrolyse choisie et on la maintient pendant une durée variant par exemple entre 8 et 12 heures.

En fin d'hydrolyse, on abaisse la température par exemple entre 20°C et 80°C.

On obtient un milieu comprenant l'acide difluoroacétique, l'eau et un sel d'ammonium dont l'anion correspond à celui de l'acide utilisé.

L'hydrolyse étant préférentiellement effectuée à l'aide d'une solution aqueuse d'acide sulfurique, le sulfate d'ammonium formé se retrouve ainsi en phase aqueuse.

On recueille l'acide difluoroacétique par tout moyen classique, par exemple par distillation.

Selon une deuxième variante de l'invention, on prépare un sel de l'acide difluoroacétique qui répond à la formule (II) dans laquelle R₁ représente un cation métallique.

On peut citer plus particulièrement de préférence un cation métallique alcalin ou alcalino-terreux.

Comme exemples plus spécifiques de cations, on peut mentionner les cations métalliques alcalins, de préférence le lithium, le sodium, le potassium ou le césium ; les cations métalliques alcalino-terreux de préférence le magnésium, le calcium, le baryum.

Dans la liste précitée, les cations métalliques préférés sont les cations sodium ou potassium.

Le procédé de préparation d'un sel d'acide difluoroacétique comprend l'hydrolyse du difluoroacétonitrile obtenu en milieu basique.

La quantité d'eau mise en oeuvre à cette étape d'hydrolyse est généralement telle que le rapport entre le nombre de moles d'eau et le nombre de moles de difluoroacétonitrile est compris entre 2 et 5.

On choisit la base apportant le cation R₁, et l'on peut citer plus particulièrement les hydroxydes des métaux alcalins ou alcalino-terreux.

On choisit préférentiellement l'hydroxyde de sodium ou de potassium.

On fait appel notamment aux solutions de soude ou de potasse ayant une concentration variant entre 30 et 50 % en masse.

La quantité de base mise en oeuvre préférentiellement est telle que le rapport entre le nombre de moles de base exprimé en ions OH⁻ et le nombre de difluoroacétonitrile varie entre 1 et 10, de préférence entre 2 et 5.

L'opération d'hydrolyse est avantageusement conduite à une température comprise entre 100°C et 130°C, pendant une durée variant par exemple entre 4 et 12 heures, de préférence entre 6 et 10 heures.

On travaille généralement à pression atmosphérique mais sous pression autogène des réactifs.

On peut également établir une atmosphère inerte, de préférence une atmosphère d'azote.

Selon un mode de réalisation pratique de l'invention, on mélange le difluoroacétonitrile et la solution basique.

On porte le mélange réactionnel à la température d'hydrolyse choisie et on la maintient pendant une durée variant par exemple entre 8 et 12 heures.

En fin d'hydrolyse, on abaisse la pression jusqu'à la pression atmosphérique.

Le milieu obtenu comprend le sel de l'acide difluoroacétique et l'ammoniac en solution : ce dernier pouvant étre éliminé notamment par entraînement à l'air ou à l'azote.

On peut récupérer le sel de l'acide difluoroacétique, à partir du mélange réactionnel notamment par la technique de séparation décrite dans WO 2010/03986.

Un autre mode de préparation d'un sel de l'acide difluoroacétique comprend la réaction d'un halogénodifluorométhane et d'une source d'anions cyanure, en présence de la quantité de base nécessaire à la fois pour effectuer la réaction de cyanation et la réaction d'hydrolyse.

Les réactifs mis en oeuvre sont tels que décrits précédemment à l'étape de préparation du difluoroacétonitrile.

La quantité de source de cyanure introduite est choisie d'une manière préférentielle de telle sorte que le rapport entre le nombre de moles de source de cyanure exprimée en CN⁻ et le nombre de moles d'halogénodifluorométhane varie entre 0,8 et 2,0 et de préférence entre 0,9 et 1,0.

La quantité de base mise en oeuvre préférentiellement est telle que le rapport entre le nombre de moles de base exprimée en OH⁻ et le nombre de moles de chlorodifluorométhane varie entre 2 et 5, et se situe plus préférentiellement aux environs de 2.

La réaction est conduite avantageusement en milieu aqueux : l'eau étant généralement apportée par la solution basique.

La température de la réaction est choisie de préférence entre 100°C et 120°C.

Elle se déroule généralement sous pression autogène des réactifs et des produits: la pression se situant généralement entre 10 et 150 bar (absolu).

On peut éventuellement conduire la réaction sous atmosphère d'un gaz inerte.

D'un point de vue pratique, le procédé selon l'invention est simple à mettre en oeuvre.

On charge dans un autoclave la source de cyanure, la solution basique (ou base + eau). On ferme l'autoclave et l'on introduit l'halogénodifluorométhane.

On porte ensuite l'ensemble à la température réactionnelle choisie entre 120°C et 130°C que l'on maintient par exemple entre 10 et 20 heures.

En fin de réaction, on ramène la pression à la pression atmosphérique et la température à la température ambiante.

On récupère le sel de l'acide difluoroacétique en phase aqueuse.

Selon une troisième variante de l'invention, on prépare les esters de l'acide difluoroacétique qui répond à la formule (II) dans laquelle R₁ représente un groupe hydrocarboné, substitué ou non, qui peut être un groupe alkyle, cycloalkyle ou aralkyle.

Dans le cadre de l'invention, on entend par « alkyle », une chaîne hydrocarbonée linéaire ou ramifiée ayant de 1 à 15 atomes de carbone et de préférence de 1 ou 2 à 10 atomes de carbone.

Des exemples de groupes alkyle préférés sont notamment méthyle, éthyle, propyle, isopropyle, butyle, isobutyle, t-butyle.

Par « cycloalkyle », on entend un groupe hydrocarboné cyclique, monocyclique comprenant de 3 à 8 atomes de carbone, de préférence, un groupe cyclopentyle ou cyclohexyle.

Par « arylalkyle », on entend un groupe hydrocarboné, linéaire ou ramifié porteur d'un cycle aromatique monocyclique en C₇-C₁₂, de préférence, benzyle : la chaîne aliphatique comprenant 1 ou 2 atomes de carbone.

Il est à noter que dans ces groupes, un ou plusieurs atomes d'hydrogène peuvent être remplacés par un substituant (par exemple, halogène).

En particulier, la chaîne hydrocarbonée peut porter préférentiellement un ou plusieurs atomes de fluor.

Ainsi, R₁ peut représenter une groupe alkyle fluoré ou perfluoré comprenant de 1 à 10 atomes de carbone et de 1 à 21 atomes de fluor, de préférence, de 3 à 21 atomes de fluor.

Le procédé de préparation des esters de l'acide difluoroacétique comprend l'hydrolyse du difluoroacétonitrile obtenu en milieu acide et en présence d'un alcool R₁-OH.

La quantité d'eau mise en oeuvre à cette étape d'hydrolyse est généralement telle que le rapport entre le nombre de moles d'eau et le nombre de moles de difluoroacétonitrile est compris entre 1 et 5.

La quantité d'alcool mis en oeuvre est choisie préférentiellement de telle sorte que le rapport entre le nombre de moles d'alcool et le nombre de moles de difluoroacétonitrile varie entre 1 et 3, de préférence entre 1 et 2.

L'acidification est réalisée à l'aide d'un acide fort ayant un pkₐ inférieur ou égal à 2.

Le pKa est défini comme la constante de dissociation ionique du couple acide/base, lorsque l'eau est utilisée comme solvant.

On choisit un acide fort qui avantageusement ne présente pas de caractère oxydant. Ainsi, l'acide nitrique n'est pas préféré. On fait appel plus préférentiellement à l'acide sulfurique, chlorhydrique ou phosphorique.

L'acide sulfurique est choisi préférentiellement.

On fait appel avantageusement à une solution d'acide fort concentrée.

On utilise plus particulièrement les formes commerciales d'acides.

On peut citer notamment les solutions d'acide sulfurique à 95 ou 98 % en masse, d'acide chlorhydrique à 37 % en masse, d'acide phosphorique à 95 - 100 % en masse.

La quantité d'acide fort mis en oeuvre est telle que le rapport entre le nombre de moles d'acide exprimé en ions H⁺ et le nombre de difluoroacétonitrile varie entre 1 et 5, de préférence entre 1 et 2.

L'opération d'hydrolyse est avantageusement conduite à une température comprise entre 100°C et 150°C.

On travaille généralement à pression atmosphérique mais sous pression autogène des réactifs.

On peut également établir une atmosphère inerte, de préférence une atmosphère d'azote.

Selon un mode de réalisation pratique de l'invention, on mélange le difluoroacétonitrile, la solution acide et l'alcool.

On porte le mélange réactionnel à la température d'hydrolyse choisie et on la maintient pendant une durée variant par exemple entre 8 et 12 heures.

En fin d'hydrolyse, on abaisse la température par exemple entre 20°C et 80°C.

On obtient l'iminoester correspondant que l'on soumet à une opération d'hydrolyse.

La quantité d'eau mise en oeuvre à cette étape d'hydrolyse est généralement telle que le rapport entre le nombre de moles d'eau et le nombre de moles de difluoroacétonitrile est compris entre 1 et 5, de préférence entre 1 et 3.

L'hydrolyse est avantageusement effectuée à une température comprise entre 50°C et 100C.

On obtient un milieu biphasique comprenant une phase organique constituée par l'ester de l'acide difluoroacétique et une phase aqueuse comprenant de l'eau et un sel d'ammonium dont l'anion correspond à celui de l'acide utilisé.

L'hydrolyse étant préférentiellement effectuée à l'aide d'une solution aqueuse d'acide chlorhydrique, le chlorure d'ammonium formé se retrouve ainsi en phase aqueuse.

On sépare les phases aqueuse et organique selon les techniques classiques de séparation liquide/liquide par exemple décantation ou centrifugation.

On recueille l'ester de l'acide difluoroacétique qui constitue la phase organique.

Selon une quatrième variante de l'invention, on prépare le difluoroacétamide qui répond à la formule (II) dans laquelle R₁ représente un groupe amino.

Le procédé de préparation du difluoroacétamide comprend l'hydrolyse contrôlée du difluoroacétonitrile obtenu en milieu acide.

La quantité d'eau mise en oeuvre à cette étape d'hydrolyse est généralement telle que le rapport entre le nombre de moles d'eau et le nombre de moles de difluoroacétonitrile est compris entre 1 et 5.

L'acidification est réalisée à l'aide d'un acide fort ayant un pkₐ inférieur ou égal à 2.

Le pKa est défini comme la constante de dissociation ionique du couple acide/base, lorsque l'eau est utilisée comme solvant.

On choisit un acide fort qui avantageusement ne présente pas de caractère oxydant. Ainsi, l'acide nitrique n'est pas préféré. On fait appel plus préférentiellement à l'acide sulfurique, chlorhydrique ou phosphorique.

L'acide sulfurique est choisi préférentiellement.

On fait appel avantageusement à une solution d'acide fort concentrée.

On utilise plus particulièrement les formes commerciales d'acides.

On peut citer notamment les solutions d'acide sulfurique à 95 ou 98 % en masse, d'acide chlorhydrique à 37 % en masse, d'acide phosphorique à 95 - 100 % en masse.

La quantité d'acide fort mis en oeuvre est telle que le rapport entre le nombre de moles d'acide exprimé en ions H⁺ et le nombre de difluoroacétonitrile varie entre 0,05 et 1 de préférence entre 0,05 et 0,2.

L'opération d'hydrolyse est avantageusement conduite à une température comprise entre 100°C et 150°C.

On travaille généralement sous pression autogène des réactifs.

On peut également établir une atmosphère inerte, de préférence une atmosphère d'azote.

Selon un mode de réalisation pratique de l'invention, on mélange le difluoroacétonitrile et la solution acide.

On porte le mélange réactionnel à la température d'hydrolyse choisie et on la maintient pendant une durée variant par exemple entre 1 et 2 heures.

En fin d'hydrolyse, on abaisse la température par exemple entre 20°C et 80°C.

On obtient un milieu biphasique comprenant une phase aqueuse comprenant l'excès d'eau et d'acide introduit et un solide principalement constituée du difluoroacétamide.

On sépare le difluoroacétamide selon les techniques classiques de séparation solide/liquide de préférence par filtration.

Il peut éventuellement être purifié selon les techniques usuelles telles que recristallisation ou extraction.

Le procédé de l'invention est avantageusement conduit dans un appareillage susceptible de résister à la corrosion du milieu réactionnel.

A cet effet, on choisit des matériaux pour la partie en contact avec le milieu réactionnel résistant à la corrosion comme les alliages à base de molybdène, chrome, cobalt, fer, cuivre manganèse, titane, zirconium, aluminium, carbone et tungstène vendus sous les marques HASTELLOY^{®}ou les alliages de nickel, chrome, fer, manganèse additivés de cuivre et/ou molybdène commercialisés sous la dénomination INCONEL^{®} et plus particulièrement les alliages HASTELLOY C 276 ou INCONEL 600, 625 ou 718.

On peut choisir également les aciers inoxydables, tels que les aciers austénitiques [Robert H. Perry et al, Perry's Chemical Engineers' Handbook, Sixth Edition (1984), page 23-44]. et plus particulièrement les aciers inoxydables 304, 304 L, 316 ou 316 L. On met en oeuvre un acier ayant une teneur en nickel au plus de 22 % en masse, de préférence comprise entre 6 et 20 %, et plus préférentiellement comprise entre 8 et 14 %.

Les aciers 304 et 304 L ont une teneur en nickel variant entre 8 et 12 % et les aciers 316 et 316 L ont une teneur en nickel variant entre 10 et 14 %.

On choisit plus particulièrement les aciers 316 L.

On peut aussi faire appel à un appareillage constitué ou revêtu d'un composé polymérique résistant à la corrosion du milieu réactionnel. On peut citer notamment, les matériaux tels que PTFE (polytétrafluoroéthylène ou Teflon) ou PFA (résines perfluoroalkyles), polyéthylène haute densité. On ne sortira pas du cadre de l'invention à utiliser un matériau équivalent.

Comme autres matériaux susceptibles de convenir pour être en contact avec le milieu réactionnel, on peut également mentionner les aciers verrés et les dérivés du graphite.

Le procédé de l'invention peut être mis en oeuvre en continu ou en discontinu.

Il est particulièrement intéressant car c'est un procédé simple qui fait appel à un réactif courant et qui permet d'obtenir l'acide difluoroacétique, ses sels, ses esters ou amide.

On donne ci-après des exemples de réalisation de l'invention. Ces exemples sont donnés à titre illustratif et sans caractère limitatif.

Dans les exemples, on définit le rendement obtenu.

Le rendement (RR) correspond au rapport entre le nombre de moles de produit formées et le nombre de moles de substrat engagées.

### Exemple 1

### Préparation du difluoroacétonitrile.

Dans un autoclave d'un volume de 200 mL équipé d'un agitateur de type Rushton, le cyanure de sodium (2,57 g, 52,4 mmol) et la soude (0,4 g, 10,5 mmol) sont dissous dans 22 mL d'eau.

L'autoclave est fermé et le chlorodifluorométhane est ajouté sous pression (9 g, 105 mmol).

La pression obtenue à température ambiante est de 12,8 bar relatif.

La température est ensuite portée à 95°C pour une durée de deux heures, puis ramenée à une température de 40°C.

L'autoclave est ramené à pression atmosphérique.

Les gaz sont récoltés puis analysés par chromatographie en phase gazeuse.

On obtient une masse totale de 6,3 g composée de chlorodifluorométhane (3,6 g) et de difluoroacétonitrile (2,7 g).

Le rendement est de 67 %.

### Exemple 2

### Préparation du difluoroacétate de sodium.

Le difluoroacétonitrile est placé dans un autoclave de 100 mL équipé d'un agitateur de type Rushton, contenant 30 g d'une solution de soude à 30 % (230 mmol).

La température est abaissée à 5°C et le difluoroacétonitrile (m = 9,8 g, 0,127 mol) est coulé à cette température.

La température du milieu est portée à 115°C pour une durée de 13 h puis ramenée à la température ambiante.

L'autoclave est ramené à pression atmosphérique.

La concentration en difluoroacétate de sodium dans la solution aqueuse obtenue est de 11,6 % en poids (analyse par RMN ¹⁹F), ce qui correspond à une masse de 2,63 g de difluoroacétate de sodium, soit 87 % de la théorie.

### Exemple 3

### Préparation du difluoroacétate de sodium.

Dans un autoclave d'un volume de 200 mL équipé d'un agitateur de type Rushton, le cyanure de sodium (2,6 g, 0,053 mmol) et la soude (4,2 g, 0,13 mol) sont dissous dans 25 mL d'eau.

L'autoclave est fermé et le chlorodifluorométhane est introduit sous pression (9 g, 105 mmol).

La température est ensuite portée à 117°C pour une durée de 17 heures, puis ramenée à une température ambiante.

L'autoclave est ramené à pression atmosphérique.

La solution aqueuse obtenue (29 g) contient 12,5 % en poids de difluoroacétate de sodium, ce qui équivaut à 3,64 g de difluoroacétate de sodium pur, soit 54 % de la théorie.

### Exemple 4

### Préparation du difluoroacétate d'éthyle.

Un mélange d'acide sulfurique à 98 % (25,5 g, 0,259 mol), d'eau (9,35 g, 0,52 mol) et d'éthanol (9 g ;0,19 mol) est introduit dans un réacteur en verre équipé d'un agitateur.

La température est abaissée à 10°C et le difluoroacétonitrile est introduit à cette température (10 g, 0,13 mol).

La température est ensuite portée à 120°C pour une durée de 14 h, puis ramenée à la température ambiante.

Le milieu est décanté et la phase supérieure est récupérée.

On obtient 10,5 g de difluoroacétate d'éthyle pur à 87 % en poids, ce qui correspond à une masse de 9,2 g de difluoroacétate d'éthyle pur (le rendement est de 57 %).

Le difluoroacétate d'éthyle brut obtenu est purifié par distillation à pression atmosphérique.

La fraction ayant un point d'ébullition compris entre 75°C et 77°C est récoltée.

On obtient 5,5 g de difluoroacétate d'éthyle avec une pureté déterminée par par chromatographie en phase gazeuse supérieure à 98 % en poids.

## Revendications

1. Procédé de préparation de difluoroacétonitrile **caractérisé par le fait qu'**il comprend la réaction d'un halogénodifluorométhane et d'une source d'anions cyanure, en milieu basique.

2. Procédé selon la revendication 1 **caractérisé par le fait que** l'halogénodifluorométhane répond à la formule suivante :
HXCF₂ (I)
dans ladite formule, X représente un atome de chlore, de brome ou d'iode.

3. Procédé selon l'une des revendications 1 et 2 **caractérisé par le fait que** l'halogénodifluorométhane est le chlorodifluorométhane (ou R22).

4. Procédé selon la revendication 1 **caractérisé par le fait que** la source d'anions cyanure est le cyanure de sodium ou de potassium.

5. Procédé selon la revendication 1 **caractérisé par le fait que** la base est un hydroxyde de métal alcalin, de préférence de sodium ou de potassium.

6. Procédé selon la revendication 5 **caractérisé par le fait que** la base mise en oeuvre est sous forme d'une solution aqueuse d'hydroxyde de métal alcalin ayant une concentration comprise entre 5 et 50 % en poids.

7. Procédé selon l'une des revendications 1 à 6 **caractérisé par le fait que** la réaction est conduite à une température choisie entre 20°C et 150°C et de préférence comprise entre 50°C et 120°C.

8. Procédé selon l'une des revendications 1 à 7 **caractérisé par le fait que** l'on mélange la source de cyanure et la base puis l'on injecte dans le milieu réactionnel, l'halogénodifluorométhane et que l'on récupère en fin de réaction, le difluoroacétonitrile formé présent dans la phase organique.

9. Procédé de préparation de l'acide difluoroacétique, de ses sels ou esters **caractérisé par le fait qu'**il comprend une première étape de préparation de difluoroacétonitrile à partir d'un halogénodifluorométhane selon le procédé décrit précédemment dans l'une des revendications 1 à 8 suivie par une opération d'hydrolyse.

10. Procédé selon la revendication 9 **caractérisé par le fait que** l'acide difluoroacétique est préparé par hydrolyse en milieu acide, du difluoroacétonitrile obtenu.

11. Procédé selon la revendication 9 **caractérisé par le fait qu'**un sel de l'acide difluoroacétique est préparé par l'hydrolyse du difluoroacétonitrile obtenu en milieu basique.

12. Procédé selon la revendication 9 **caractérisé par le fait qu'**un ester de l'acide difluoroacétique est préparé par l'hydrolyse du difluoroacétonitrile obtenu en milieu acide et en présence d'un alcool.

13. Procédé selon la revendication 9 **caractérisé par le fait que** le difluoroacétamide est préparé par l'hydrolyse contrôlée du difluoroacétonitrile obtenu en milieu acide.

14. Procédé selon la revendication 13 **caractérisé par le fait que** la quantité d'acide fort mis en oeuvre est telle que le rapport entre le nombre de moles d'acide exprimé en ions H⁺ et le nombre de difluoroacétonitrile varie entre 0,05 et 1, de préférence entre 0,05 et 0,2.

15. Procédé de préparation d'un sel de l'acide difluoroacétique **caractérisé par le fait qu'**il comprend la réaction d'un halogénodifluorométhane et d'une source d'anions cyanure, en présence de la quantité de base nécessaire à la fois pour effectuer la réaction de cyanation et la réaction d'hydrolyse.

16. Procédé selon la revendication 15 **caractérisé par le fait que** l'halogénodifluorométhane est le chlorodifluorométhane (ou R22).

17. Procédé selon la revendication 15 **caractérisé par le fait que** la source d'anions cyanure est le cyanure de sodium ou de potassium.

18. Procédé selon la revendication 15 **caractérisé par le fait que** la base est un hydroxyde de métal alcalin, de préférence de sodium ou de potassium.

19. Procédé selon la revendication 18 **caractérisé par le fait que** la base mise en oeuvre est sous forme d'une solution aqueuse d'hydroxyde de métal alcalin ayant une concentration comprise entre 5 et 50 % en poids.

20. Procédé selon l'une des revendications 15 à 19 **caractérisé par le fait que** la quantité de base mise en oeuvre est telle que le rapport entre le nombre de moles de base exprimée en OH⁻ et le nombre de moles de chlorodifluorométhane varie entre 2 et 5, et se situe de préférence aux environs de 2.

21. Procédé selon l'une des revendications 15 à 20 **caractérisé par le fait que** la température de la réaction est choisie entre 100°C et 120°C.

## Patentansprüche

1. Verfahren zur Herstellung von Difluoracetonitril, **dadurch gekennzeichnet, dass** es die Umsetzung eines Halogendifluormethans und einer Quelle von Cyanid-Anionen in basischem Medium umfasst.

2. Verfahren nach Anspruch 1, **dadurch gekennzeichnet, dass** das Halogendifluormethan der folgenden Formel entspricht:
HXCF₂ (I)
worin X für ein Chlor-, Brom- oder Iodatom steht.

3. Verfahren nach einem der Ansprüche 1 und 2, **dadurch gekennzeichnet, dass** es sich bei dem Halogendifluormethan um Chlordifluormethan (oder R22) handelt.

4. Verfahren nach Anspruch 1, **dadurch gekennzeichnet, dass** es sich bei der Quelle von Cyanid-Anionen um Natrium- oder Kaliumcyanid handelt.

5. Verfahren nach Anspruch 1, **dadurch gekennzeichnet, dass** es sich bei der Base um ein Alkalimetallhydroxid, vorzugsweise Natrium- oder Kaliumhydroxid, handelt.

6. Verfahren nach Anspruch 5, **dadurch gekennzeichnet, dass** die Base in Form einer wässrigen Lösung von Alkalimetallhydroxid mit einer Konzentration zwischen 5 und 50 Gew.-% durchgeführt wird.

7. Verfahren nach einem der Ansprüche 1 bis 6, **dadurch gekennzeichnet, dass** die Umsetzung bei einer Temperatur zwischen 20°C und 150°C und vorzugsweise zwischen 50°C und 120°C durchgeführt wird.

8. Verfahren nach einem der Ansprüche 1 bis 7, **dadurch gekennzeichnet, dass** man die Cyanid-Quelle und die Base mischt und dann das Halogen-difluormethan in das Reaktionsmedium einleitet und am Ende der Umsetzung das gebildete, in der organischen Phase vorliegende Difluoracetonitril gewinnt.

9. Verfahren zur Herstellung von Difluoressigsäure, Salzen davon oder Estern davon, **dadurch gekennzeichnet, dass** es einen ersten Schritt der Herstellung von Difluoracetonitril aus einem Halogendifluormethan gemäß dem oben in einem der Ansprüche 1 bis 8 beschriebenen Verfahren gefolgt von einem Hydrolysevorgang umfasst.

10. Verfahren nach Anspruch 9, **dadurch gekennzeichnet, dass** die Difluoressigsäure durch Hydrolyse des erhaltenen Difluoracetonitrils in saurem Medium hergestellt wird.

11. Verfahren nach Anspruch 9, **dadurch gekennzeichnet, dass** durch Hydrolyse des erhaltenen Difluoracetonitrils in basischem Medium ein Difluoressigsäuresalz hergestellt wird.

12. Verfahren nach Anspruch 9, **dadurch gekennzeichnet, dass** durch Hydrolyse des erhaltenen Difluoracetonitrils in saurem Medium und in Gegenwart eines Alkohols ein Ester von Difluoressigsäure hergestellt wird.

13. Verfahren nach Anspruch 9, **dadurch gekennzeichnet, dass** das Difluoracetamid durch kontrollierte Hydrolyse des erhaltenen Difluoracetonitrils in saurem Medium hergestellt wird.

14. Verfahren nach Anspruch 13, **dadurch gekennzeichnet, dass** eine solche Menge an starker Säure verwendet wird, dass das Verhältnis zwischen der Zahl der Mole Säure, ausgedrückt als H⁺-Ionen, und der Zahl von Difluoracetonitril zwischen 0,05 und 1, vorzugsweise zwischen 0,05 und 0,2, variiert.

15. Verfahren zur Herstellung eines Salzes von Difluoressigsäure, **dadurch gekennzeichnet, dass** es die Umsetzung eines Halogendifluormethans und einer Quelle von Cyanid-Anionen in Gegenwart der für die Durchführung sowohl der Cyanierungsreaktion als auch der Hydrolysereaktion benötigten Basenmenge umfasst.

16. Verfahren nach Anspruch 15, **dadurch gekennzeichnet, dass** es sich bei dem Halogendifluormethan um Chlordifluormethan (oder R22) handelt.

17. Verfahren nach Anspruch 15, **dadurch gekennzeichnet, dass** es sich bei der Quelle von Cyanid-Anionen um Natrium- oder Kaliumcyanid handelt.

18. Verfahren nach Anspruch 15, **dadurch gekennzeichnet, dass** es sich bei der Base um ein Alkalimetallhydroxid, vorzugweise Natrium- oder Kaliumhydroxid, handelt.

19. Verfahren nach Anspruch 18, **dadurch gekennzeichnet, dass** die Base in Form einer wässrigen Lösung von Alkalimetallhydroxid mit einer Konzentration zwischen 5 und 50 Gew.-% durchgeführt wird.

20. Verfahren nach einem der Ansprüche 15 bis 19, **dadurch gekennzeichnet, dass** die Base in einer solchen Menge verwendet wird, dass das Verhältnis zwischen der Zahl der Mole Base, ausgedrückt als OH⁻-Ionen, und der Zahl der Mole Chlordifluormethan zwischen 2 und 5 variiert und vorzugsweise bei ungefähr 2 liegt.

21. Verfahren nach einem der Ansprüche 15 bis 20, **dadurch gekennzeichnet, dass** die Temperatur der Umsetzung zwischen 100°C und 120°C gewählt wird.

## Claims

1. Process for preparing difluoroacetonitrile, **characterized in that** it comprises the reaction of a halodifluoromethane and of a source of cyanide anions, in a basic medium.

2. Process according to Claim 1, **characterized in that** the halodifluoromethane corresponds to the following formula:
HXCF₂ (I)
in said formula, X represents a chlorine, bromine or iodine atom.

3. Process according to either of Claims 1 and 2, **characterized in that** the halodifluoromethane is chlorodifluoromethane (or R22).

4. Process according to Claim 1, **characterized in that** the source of cyanide anions is sodium cyanide or potassium cyanide.

5. Process according to Claim 1, **characterized in that** the base is an alkali metal hydroxide, preferably sodium hydroxide or potassium hydroxide.

6. Process according to Claim 5, **characterized in that** the base used is in the form of an aqueous solution of alkali metal hydroxide having a concentration between 5% and 50% by weight.

7. Process according to one of Claims 1 to 6, **characterized in that** the reaction is performed at a temperature chosen to be between 20°C and 150°C and preferably between 50°C and 120°C.

8. Process according to one of Claims 1 to 7, **characterized in that** the cyanide source and the base are mixed, then the halodifluoromethane is injected into the reaction medium, and that, at the end of the reaction, the difluoroacetonitrile formed, present in the organic phase, is recovered.

9. Process for preparing difluoroacetic acid, salts thereof or esters thereof, **characterized in that** it comprises a first step of preparing difluoroacetonitrile from a halodifluoromethane according to the process described previously in one of Claims 1 to 8, followed by a hydrolysis operation.

10. Process according to Claim 9, **characterized in that** the difluoroacetic acid is prepared by hydrolysis, in an acid medium, of the difluoroacetonitrile obtained.

11. Process according to Claim 9, **characterized in that** a salt of difluoroacetic acid is prepared by hydrolysis of the difluoroacetonitrile obtained, in a basic medium.

12. Process according to Claim 9, **characterized in that** an ester of difluoroacetic acid is prepared by hydrolysis of the difluoroacetonitrile obtained, in an acid medium and in the presence of an alcohol.

13. Process according to Claim 9, **characterized in that** difluoroacetamide is prepared by controlled hydrolysis of the difluoroacetonitrile obtained, in an acid medium.

14. Process according to Claim 13, **characterized in that** the amount of strong acid used is such that the ratio between the number of moles of acid expressed as H⁺ ions and the number of difluoroacetonitrile varies between 0.05 and 1, preferably between 0.05 and 0.2.

15. Process for preparing a salt of difluoroacetic acid, **characterized in that** it comprises the reaction of a halodifluoromethane and of a source of cyanide anions, in the presence of the amount of base necessary both for carrying out the cyanation reaction and the hydrolysis reaction.

16. Process according to Claim 15, **characterized in that** the halodifluoromethane is chlorodifluoromethane (or R22).

17. Process according to Claim 15, **characterized in that** the source of cyanide anions is sodium cyanide or potassium cyanide.

18. Process according to Claim 15, **characterized in that** the base is an alkali metal hydroxide, preferably sodium hydroxide or potassium hydroxide.

19. Process according to Claim 18, **characterized in that** the base used is in the form of an aqueous solution of alkali metal hydroxide having a concentration between 5% and 50% by weight.

20. Process according to one of Claims 15 to 19, **characterized in that** the amount of base used is such that the ratio between the number of moles of base expressed as OH⁻ and the number of moles of chlorodifluoromethane varies between 2 and 5, and preferably lies close to 2.

21. Process according to one of Claims 15 to 20, **characterized in that** the temperature of the reaction is chosen to be between 100°C and 120°C.
